# NOUVEAU FASCICULE DE BREVET EUROPEEN

(11) **EP 0 645 999 B2**
(45) Date de publication et mention de la décision concernant l'opposition: **27.09.2006**
(45) Mention de la délivrance du brevet: 31.01.1996
(21) Numéro de dépôt: 93913170.2
(22) Date de dépôt: 22.06.1993
(51) Int. Cl.: A61Q 5/10, A61K 8/22

(54) **PROCEDE DE TEINTURE DES FIBRES KERATINIQUES AVEC DES DERIVES INDOLIQUES OU INDOLINIQUES, DU PEROXYDE D'HYDROGENE ET UNE PEROXYDASE**
VERFAHREN ZUR FAERBUNG VON KERAMISCHEN FASERN MIT INDOL ODER INDOLDERIVATEN, WASSERSTOFFPEROXYD UND EINER PEROXYDASE
METHOD FOR DYEING KERATIN FIBRES WITH INDOLE OR INDOLIN DERIVATIVES, HYDROGEN PEROXIDE AND A PEROXIDASE

(30) Priorité: 25.06.1992 FR 9207784
(43) Date de publication de la demande: 05.04.1995
(73) Titulaire: L'ORÉAL, 75008 Paris (FR)
(72) Inventeur: SAMAIN, Henri, F-91570 Bièvres (FR); DUBIEF, Claude, F-78150 Le Chesnay (FR)
(74) Mandataire: Casalonga, Axel
(86) Numéro de dépôt international: PCT/FR1993/000617
(87) Numéro de publication internationale: WO 1994/000100

(56) Documents cités:
- EP-A- 0 441 689
- WO-A-91/17739
- DE-A- 2 155 339
- DE-A- 3 824 122
- FR-A- 2 112 550
- FR-A- 2 252 841
- US-A- 4 515 773

## Description

L'invention concerne un procédé de teinture des fibres kératiniques mettant en oeuvre des dérivés indoliques ou indoliniques, du peroxyde d'hydrogène et une enzyme-peroxydase.

La coloration des cheveux dite "permanente" consiste à obtenir une coloration visible sur les cheveux pendant plusieurs semaines, résistante aux shampooings.

Pour cela, on peut utiliser des précurseurs indoliques ou indoliniques susceptibles de s'oxyder en présence d'un agent oxydant pour donner des produits colorés, dont la structure chimique est voisine des pigments capillaires tels que la mélanine.

L'agent oxydant couramment utilisé est le peroxyde d'hydrogène.

Les précurseurs de colorants d'oxydation choisis parmi les dérivés indoliques ou indoliniques ont l'avantage de produire des colorations proches des nuances naturelles.

Les procédés de teinture par oxydation de l'état de la technique mettant en oeuvre des précurseurs du type indolique ou indolinique et le peroxyde d'hydrogène comme système d'oxydant, sont généralement effectués dans des milieux de teinture alcalins pour obtenir des propriétés tinctoriales satisfaisantes. Cependant, l'application de ces milieux alcalins entraîne des dégradations au niveau des fibres.

Le document FR-A-2 252 841 décrit un procédé de teinture pour cheveux.

La demanderesse vient de découvrir dune manière surprenante un nouveau procédé de teinture par oxydation des fibres kératiniques à base de dérivés indoliques ou indoliniques qui, après oxydation par le peroxyde d'hydrogène sous forme d'eau oxygénée ou produit par une source naturelle enzymatique, en présence d'une enzyme peroxydase, permettait d'obtenir dans des conditions de pH doux, des colorations puissantes, résistantes aux shampooings, sans dégradation des fibres.

L'invention a donc pour objet un nouveau procédé de teinture mettant en oeuvre au moins un dérivé indolique ou indolinique, du peroxyde d'hydrogène et une enzyme-peroxydase.

Un autre objet de l'invention est constitué par les compositions et dispositifs à plusieurs compartiments utilisés dans le cadre de ce procédé.

D'autres objets apparaîtront à la lecture de la description et des exemples qui suivent.

Le procédé de teinture des fibres kératiniques, et en particulier des cheveux, conforme à l'invention, est caractérisé par le fait que l'on applique sur ces fibres, au moins :
- un composant (1) contenant dans un milieu aqueux approprié pour la teinture, au moins un composé de formule : 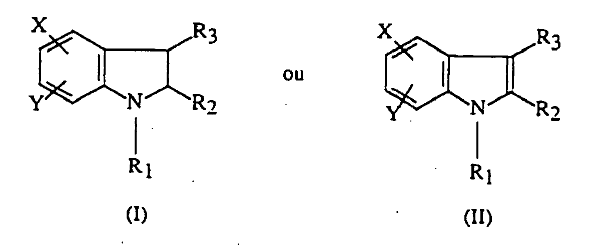 dans lesquelles :
   R₁ représente un atome d'hydrogène ou un radical alkyle en C₁-C₄;
   R₂ représente un atome d'hydrogène, un radical alkyle en C₁-C₄ ou -COOH;
   R₃ représente un atome d'hydrogène ou un radical alkyle en C₁-C₄ ;
   X désigne un atome d'hydrogène. NH₂, OH, un radical alkyle en C₁-C₄ ou un radical alcoxy en C₁-C₄;
   Y désigne OH ou NH₂ ;
   sous réserve que lorsque X désigne OH ou alkyle, X occupe les positions 5, 6 ou 7 et X est en position ortho par rapport à Y ;
- un composant (2) contenant dans un milieu aqueux approprié pour la teinture, au moins une enzyme-peroxydase ;
- un composant (3) contenant dans un milieu aqueux approprié pour la teinture, au moins du peroxyde d'hydrogène ou une source de peroxyde d'hydrogène enzymatique ;

le composant (1) étant appliqué sur les fibres en premier, séparément du composant (3),
dans lequel on applique
dans une première étape une composition contenant dans un milieu aqueux approprié pour la teinture, au moins le composant (1) et éventuellement le composant (2), puis
- soit dans une deuxième étape une composition contenant dans un milieu aqueux approprié pour la teinture au moins les composants (2) et (3), la composition contenant le composant (1) ne contenant pas le composant (2),
- soit dans une deuxième étape une composition contenant dans un milieu aqueux approprié pour la teinture le composant (2) et dans une troisième étape une composition contenant dans un milieu aqueux approprié pour la teinture le composant (3), la composition contenant le composant (1) ne contenant pas le composant (2)
- soit dans une deuxième étape une composition contenant dans un milieu aqueux approprié pour la teinture le composant (3) lorsque la composition appliquée dans la première étape contient le composant (2),
le pH des compositions mises en oeuvre variant de 4,5 à 7,5.

Parmi les précurseurs indoliques ou indoliniques de formule (I) ou (II) préférentiels, on peut citer le 5,6-dihydroxyindole, le 2-méthyl 5,6-dihydroxyindole, le 3-méthyl 5,6-dihydroxyindole, le 4-hydroxyindole, le 2,3-diméthyl 5,6-dihydroxyindole, le 6-hydroxy 5-méthoxyindole, le 6-hydroxyindole, le 5-hydroxyindole, le 7-hydroxyindole, le 7-aminoindole, le 5-aminoindole, l'acide 5,6-dihydroxyindole 2-carboxylique, le 4-aminoindole, le 1-méthyl 5,6-dihydroxyindole, la 5,6-dihydroxyindoline, la N-méthyl 5,6-dihydroxyindoline; la N-éthyl 5,6-dihydroxyindoline, la N-butyl 5,6-dihydroxyindoline, la 4-hydroxy 5-méthoxyindoline, la 6-hydroxy 7-méthoxyindoline, la 6,7-dihydroxyindoline, la 4,5-dihydroxyindoline, la 5-méthoxy 6-hydroxyindoline et leurs sels.

L'enzyme-peroxydase utilisée conformément à l'invention, est une enzyme codée 1.11.1. selon le système de classement des enzymes (Enzyme Nomenclature, 1984, Academic Press Inc.).

On peut citer à titre d'exemple, les peroxydases provenant de sources naturelles telles que le raifort, le navet, la figue, le lait de vache ou le soja. Elles sont mises en oeuvre selon l'invention à différents degrés de purification et à des quantités variant en fonction du degré de pureté.

On utilise notamment les peroxydases issues du raifort telles que les produits vendus par la Société SIGMA.

Les peroxydases conformes à la présente invention sont utilisées dans des quantités comprises entre 100 et 40.000 unités catalytiques de peroxydase pour 100 g de composants (1), (2) et (3) tels que définis ci-dessus, à appliquer sur les cheveux; les unités catalytiques étant déterminées au cours de la réaction d'oxydation du pyrogallol par le peroxyde d'hydrogène à pH 6 et 20°C (une unité catalytique permet de transformer en 20 secondes 1 mg de pyrogallol en purpurogallin).

Dans le cas où l'on utilise comme agent oxydant du peroxyde d'hydrogène sous forme de solution d'eau oxygénée, les quantités de peroxydase sont de préférence comprises entre 1000 et 20.000 unités catalytiques.

Les sources de peroxyde d'hydrogène enzymatiques conformes à la présente invention, sont constituées d'un ou plusieurs substrat(s) naturel(s) associé(s) à un oudes système(s) enzymatique(s) du type oxydase catalysant l'oxydation d'une molécule de substrat par une molécule d'oxygène par transfert d'un seul atome d'oxygène.

Parmi les substrats naturels utilisés, on peut citer le glucose, l'acide urique, les alcools, les acides aminés, l'acide lactique. Ils sont utilisés à des concentrations comprises de préférence entre 1 et 25% en poids et plus particulièrement entre 3 et 15% en poids par rapport au poids total du ou des composants (2) et/ou (3).

Les systèmes enzymatiques du type oxydase selon l'invention sont choisis parmi les oxydases codées 1.x.3, catalysant l'oxydation d'une molécule de substrat par une molécule d'oxygène par transfert d'un seul atome de la molécule d'oxygène. Les quantités utilisées sont déterminées en fonction de leur pureté. Elles varient de préférence entre 100 et 20.000 unités catalytiques d'oxydase pour 100 g de composants (1), (2) et (3) à appliquer sur les fibres kératiniques; les unités étant déterminées dans la réaction d'oxydation à l'air du substrat à pH 5,1 et 37°C (une unité d'oxydase permet de transformer en 1 minute 1 µg de substrat).

Parmi les systèmes substrat naturel/enzyme d'oxydation du substrat par l'oxygène par transfert d'un seul atome d'oxygène, on peut citer :
- glucose/glucose oxydase
- galactose/galactose oxydase
- pyranose/pyranose oxydase
- L-sorbose/L-sorbose oxydase
- éthanol/alcool oxydase (1.1.3.13)
- isopropanol/alcool secondaire oxydase (1.1.3.18)
- acide pyruvique/pyruvate oxydase
- acide oxalique/oxalate oxydase
- acide aspartique/aspartate oxydase
- acide glutamique/L-glutamate oxydase
- acide urique/uricase
- acide lactique/lactate oxydase,
ou leurs mélanges.

Une forme de réalisation de l'invention consiste à appliquer sur les fibres kératiniques, dans un premier temps, une composition (A) contenant dans un milieu aqueux approprié pour la teinture, le composant (1) tel que défini ci-dessus, et dans une seconde étape, une composition (B) contenant dans un milieu aqueux approprié pour la teinture, au moins les composants (2) et (3) tels que définis ci-dessus.

Un rinçage peut être effectué entre les deux applications.

Le temps de pose de la première composition (A) varie de préférence entre 20 secondes et 20 minutes lorsque le composant (3) contient une solution aqueuse de peroxyde d'hydrogène ou de 1 minute à 1 heure lorsque le composant (3) contient une source de peroxyde d'hydrogène enzymatique et celui de la deuxième composition (B) entre 1 minute et 1 heure et de plus particulièrement entre 5 minutes et 20 minutes.

Une deuxième forme de réalisation de l'invention consiste à appliquer successivement sur les fibres une première composition (C) contenant les composants (1) et (2), puis une deuxième composition (D) contenant dans un milieu approprié pour la teinture, le composant (3). Le temps de pose de la composition (C) varie de préférence entre 20 secondes et 20 minutes, celui de la composition (D) entre 1 minute et 1 heure et plus particulièrement entre 5 et 20 minutes.

Une troisième forme de réalisation de l'invention consiste à appliquer successivement sur les fibres, une composition (E) contenant le composant (1), une composition (F) contenant le composant (2) et une composition (G) contenant le composant (3).

Les temps de pose des compositions (E) et (F) varient de préférence entre 20 secondes et 20 minutes, celui de la composition (G) varie de préférence entre 1 minute et 1 heure et plus particulièrement entre 5 et 20 minutes.

Selon les différentes formes de procédés de teinture de l'invention, un rinçage final et un séchage peuvent être effectués à la fin du traitement.

Les agents alcalinisants utilisables dans ces compositions peuvent être en particulier des amines telles que des alcanolamines, des alkylamines, des hydroxydes ou des carbonates alcalins ou d'ammonium.

Les agents d'acidification utilisables dans les compositions de l'invention peuvent être choisis parmi l'acide lactique, l'acide acétique, l'acide tartrique; l'acide phosphorique, l'acide chlorhydrique et l'acide citrique.

Les compositions tinctoriales (A), (C) et (E) telles que définies précédemment, contiennent les dérivés indoliques ou indoliniques de formule (I) ou (II) dans des concentrations comprises de préférence entre 0,2 et 5% en poids par rapport au poids total de chacune de ces compositions.

Elles peuvent contenir en plus un cosolvant choisi parmi les alcools inférieurs tels que l'alcool éthylique ou l'isopropanol; les éthers de glycol comme les éthers monométhytique, monoéthylique ou monobutylique de l'éthylèneglycol ou du diéthylèneglycol, les monométhyléthers du propylèneglycol et du dipropylèneglycol; les esters de glycol comme l'acétate de monométhyléther ou de monoéthyléther de l'éthylèneglycol; les glycols comme l'éthylèneglycol, le propylèneglycol; les esters inférieurs comme le lactate de méthyle.

Les solvants préférés sont l'alcool éthylique et le propylèneglycol.

Les solvants sont présents dans des concentrations de préférence comprises entre 0.5 et 75% et en particulier entre 2 et 50% en poids par rapport au poids total de la composition.

Les compositions tinctoriales selon l'invention peuvent contenir d'autres colorants habituellement utilisés pour la teinture des fibres kératiniques, notamment des colorants directs tels que les dérivés nitrés benzéniques, les colorants azoïques, les anthraquinones, les naphtoquinones et benzoquinones ou les colorants d'oxydation du type para ou ortho et/ou des coupleurs.

Les compositions oxydantes de l'invention telles que les compositions (B), (D) ou (G) telles que définies ci-dessus, contiennent le peroxyde d'hydrogène dans des quantités comprises entre 0,03 et 10% et de préférence entre 0,05 et 5% en poids par rapport au poids total de l'une de ces compositions.

Les compositions mises en oeuvre dans les différents procédés de teinture de l'invention peuvent contenir en outre différents adjuvants tels que les agents tensio-actifs anioniques, cationiques, non-ioniques, amphotères ou leurs mélanges, des agents épaississants, des parfums, des agents séquestrants, des agents de traitement des fibres kératiniques, les agents dispersants, les agents de conditionnement, des agents de gonflement des fibres kératiniques, des agents conservateurs.

Les compositions mises en oeuvre dans les différents procédés de teinture de l'invention, peuvent se présenter sous forme de lotions plus ou moins épaissies, de gels ou d'émulsions. '

En vue de la mise en oeuvre des différentes formes de procédé de teinture selon l'invention, les compositions peuvent être conditionnées dans des dispositifs à plusieurs compartiments encore appelés "kit" ou "nécessaire de teinture", comportant tous les composants (1), (2) et (3) destinés à être appliqués pour une même teinture sur des fibres kératiniques.

Selon un mode de réalisation, le kit ou nécessaire de teinture comporte un premier compartiment renfermant une composition (A) telle que définie précédemment et un second compartiment contenant une composition (B) telle que définie précédemment.

Un autre mode de réalisation consiste en un kit de teinture comportant dans un premier compartiment, une composition (C) et dans un second compartiment, une composition (D) telle que définie précédemment.

Un autre mode de réalisation consiste en un kit de teinture comprenant trois compartiments dont un une composition (E), le deuxième une composition (F) et le troisième une composition (G).

Les exemples suivants sont destinés à illustrer l'invention sans toutefois présenter un caractère limitatif.

### EXEMPLES DE COMPOSITIONS

### EXEMPLE 1

### Composition (A1)

| | |
|---|---|
| - 5.6-dihydroxyindole | 1 g |
| - Alcool éthylique | 10 g |
| - Eau déminéralisée | qsp 100 g |
| Le pH de la solution est de 6,4. | |

### Composition (B1)

| | |
|---|---|
| - peroxydase de raifort | 2600 unités |
| - Eau oxygénée à 20 volumes (acidifiée à pH 2 par l'acide orthophosphorique) | 2,5 g |
| - Monoéthanolamine qs pH = 5,1 | |
| - Eau déminéralisée | qsp 100 g |

La première composition est appliquée sur les cheveux et laissée posée pendant 15 minutes. On rince. On applique la deuxième composition en laissant poser pendant 15 minutes. On rince. On note après shampooing que les cheveux, initialement blancs, sont de couleur châtain. La teinte acquise résiste aisément à plusieurs shampooings.

### EXEMPLE 2

### Composition (A2)

| | |
|---|---|
| - 5,6-dihydroxyindole | 0.5 g |
| - Alcool éthylique | 10 g |
| - Eau déminéralisée | qsp 100 g |
| Le pH de la solution est de 6,5. | |

### Composition (B2)

| | |
|---|---|
| - Peroxydase de raifort | 1300 unités |
| - Eau oxygénée à 20 volumes (acidifiée à pH 2 par l'acide orthophosphorique) | 62 g |
| - Monoéthanolamine qs pH = 5,5 | |
| - Eau déminéralisée | qsp 100 g |

Le même procédé que pour l'exemple 1 est utilisé. On note après shampooing que les cheveux, initialement blancs, sont de couleur châtain clair.

### EXEMPLE 3

### Composition (C3)

| | |
|---|---|
| - 5,6-dihydroxyindole | 1,5 g |
| - Peroxydase de raifort | 3000 unités |
| - Alcool éthylique | 10 g |
| - Eau déminéralisée | qsp 100 g |
| Le pH de la solution est de 6.5. | |

### Composition (D3)

| | |
|---|---|
| - Eau oxygénée à 20 volumes (acidifiée à pH 2 par l'acide orthophosphorique) | 12 g |
| - Monoéthanolamine qs pH = 6 | |
| - Eau déminéralisée | qsp 100 g |

La premi ère composition est appliquée sur les cheveux et laissée posée pendant 5 minutes. Sans rincer, on applique la deuxième composition en laissant poser pendant 20 minutes. On note après shampooing que les cheveux, initialement blancs, sont de couleur châtain.

### EXEMPLE 4

### Composition (E4)

| | |
|---|---|
| - 5,6-dihydroxyindole | 1 g |
| - Alcool éthylique | 10 g |
| - Eau déminéralisée | qsp 100 g |
| Le pH de la solution est de 6,5. | |

### Composition (F4)

| | |
|---|---|
| - Peroxydase de raifort | 3000 unités |
| - Eau déminéralisée | qsp 100 g |

### Composition (G4)

| | |
|---|---|
| - Eau oxygénée à 20 volumes (acidifiée à pH 2 par l'acide orthophosphorique) | 2,5 g |
| - Monoéthanolamine qs pH = 6 | |
| - Eau déminéralisée | qsp 100 g |

La première composition est appliquée sur les cheveux et laissée posée pendant 5 minutes. On rince les cheveux. La deuxième composition est appliquée sur les cheveux et laissée posée pendant 5 minutes. On applique la troisième composition en laissant poser 15 minutes. On rince. On note après shampooing que les cheveux, initialement blancs, sont de couleur châtain.

### EXEMPLE 5

### Composition (A5)

| | |
|---|---|
| - Bromhydrate de 2-méthyl 5,6-dihydroxyindole | 1 g |
| - Alcool éthylique | 10 g |
| - Triéthanolamine qs pH = 7 | |
| - Eau déminéralisée | qsp 100 g |

### Composition (B5)

| | |
|---|---|
| - Peroxydase de raifort | 2600 unités |
| - Eau oxygénée à 20 volumes (acidifiée à pH 2 par l'acide orthophosphorique) | 2,5 g |
| - Triéthanolamine qs pH = 5,5 | |
| - Eau déminéralisée | qsp 100 g |

Le même procédé qu dans l'exemple 1 est utilisé. Une coloration châtain clair est obtenue sur cheveux initialement blancs.

### EXEMPLE 6

### Composition (A6)

| | |
|---|---|
| - 3-méthyl 5,6-dihydroxyindole | 1 g |
| - Alcool éthylique | 10 g |
| - Conservateur qs | |
| - Triéthanolamine qs pH = 6,8 | |
| - Eau déminéralisée | qsp 100 g |

### Composition (B6)

| | |
|---|---|
| - Peroxydase de raifort | 2600 unités |
| - Eau oxygénée à 20 volumes (acidifiée à pH 2 par l'acide orthophosphorique) | 2,5 g |
| - Monoéthanolamine qs pH = 5,5 | |
| - Eau déminéralisée | qsp 100 g |

On utilise le même procédé que dans l'exemple 1. Une coloration châtain est obtenue sur cheveux initialement blancs.

### EXEMPLE 7

### Composition (A7)

| | |
|---|---|
| - 4-hydroxyindole | 1 g |
| - Alcool éthylique | 10 g |
| - Ammoniaque à 20% qs pH = 7 | |
| - Eau déminéralisée | qsp 100 g |

### Composition (B7)

| | |
|---|---|
| - Peroxydase de raifort | 1600 unités |
| - Eau oxygénée à 20 volumes (acidifiée à pH 2 par l'acide orthophosphorique) | 2,5 g |
| - Monoéthanolamine qs pH = 5,5 | |
| - Eau déminéralisée | qsp 100 g |

On utilise le même procédé que dans l'exemple 1. Une coloration châtain est obtenue sur cheveux initialement blancs.

### EXEMPLE 8

### Composition (A8)

| | |
|---|---|
| - Bromhydrate de 2,3-diméthyl 5,6-dihydroxyindole | 1 g |
| - Alcool éthylique | 10 g |
| - Triéthanolamine qs pH = 7 | |
| - Eau déminéralisée | qsp 100 g |

### Composition (B8)

| | |
|---|---|
| - Peroxydase de raifort | 2600 unités |
| - Eau oxygénée à 20 volumes (acidifiée à pH 2 par l'acide orthophosphorique) | 2,5 g |
| - Monoéthanolamine qs pH = 5,5 | |
| - Eau déminéralisée | qsp 100 g |

On utilise le même procédé que dans l'exemple 1. Une coloration châtain clair est obtenue sur cheveux initialement blancs.

### EXEMPLE 9

### Composition (A9)

| | |
|---|---|
| - 6-hydroxy 5-méthoxyindole | 1 g |
| - Alcool éthylique | 10 g |
| - Triéthanolamine qs pH = 5,5 | |
| - Eau déminéralisée | qsp 100 g |

### Composition (B9)

| | |
|---|---|
| - Peroxydase de raifort | 2600 unités |
| - Eau oxygénée à 20 volumes (acidifiée à pH 2 par l'acide orthophosphorique) | 2,5 g |
| - Monoéthanolamine qs pH = 5,5 | |
| - Eau déminéralisée | qsp 100 g |

On utilise le même procédé que dans l'exemple 1. Une coloration châtain clair est obtenue sur cheveux initialement blancs.

### EXEMPLE 10

### Composition (A10)

| | |
|---|---|
| - 6-hydroxyindole | 1 g |
| - Alcool éthylique | 10 g |
| - Triéthanolamine qs pH = 7 | |
| - Eau déminéralisée | qsp 100 g |

### Composition (B10)

| | |
|---|---|
| - Peroxydase de raifort | 2600 unités |
| - Eau oxygénée à 20 volumes (acidifiée à pH 2 par l'acide orthophosphorique) | 2,5 g |
| - Monoéthanolamine qs pH = 5,5 | |
| - Eau déminéralisée | qsp 100 g |

On utilise le même procédé que dans l'exempte 1. Une coloration châtain est obtenue sur cheveux initialement blancs.

### EXEMPLE 11

### Composition (A11)

| | |
|---|---|
| - 5-hydroxyindole | 1 g |
| - Alcool éthylique | 10 g |
| - Triéthanolamine qs pH = 6,8 | |
| - Eau déminéralisée | qsp 100 g |

### Composition (B11)

| | |
|---|---|
| - Peroxydase de raifort | 2600 unités |
| - Eau oxygénée à 20 volumes (acidifiée à pH 2 par l'acide orthophosphorique) | 2,5 g |
| - Monoéthanolamine qs pH = 5,5 | |
| - Eau déminéralisée | qsp 100 g |

On utilise le même procédé que dans l'exemple 1. Une coloration blond foncé est obtenue sur cheveux initialement blancs.

### EXEMPLE 12

### Composition (A12)

| | |
|---|---|
| - 7-hydroxyindole | 1 g |
| - Alcool éthylique | 10 g |
| - Triéthanolamine qs pH = 7 | |
| - Eau déminéralisée | qsp 100 g |

### Composition (B12)

| | |
|---|---|
| - Peroxydase de raifort | 3600 unités |
| - Eau oxygénée à 20 volumes (acidifiée à pH 2 par l'acide orthophosphorique) | 2,5 g |
| - Monoéthanolamine qs pH = 5,5 | |
| - Eau déminéralisée | qsp 100 g |

On utilise le même procédé que dans l'exemple 1. Une coloration châtain irisée est obtenue sur cheveux initialement blancs.

### EXEMPLE 13

### Composition (A13)

| | |
|---|---|
| - 7-aminoindole | 1 g |
| - Alcool éthylique | 10 g |
| - Triéthanolamine qs pH = 5,6 | |
| - Eau déminéralisée | qsp 100 g |

### Composition (B13)

| | |
|---|---|
| - Peroxydase de raifort | 2600 unités |
| - Eau oxygénée à 20 volumes (acidifiée à pH 2 par l'acide orthophosphorique) | 2,5 g |
| - Monoéthanolamine qs pH = 5,5 | |
| - Eau déminéralisée | qsp 100 g |

On utilise le même procédé que dans l'exemple 1. Une coloration châtain clair est obtenue sur cheveux initialement blancs.

### EXEMPLE 14

### Composition (A14)

| | |
|---|---|
| - Acide 5,6-dihydroxyindole 2-carboxylique | 1 g |
| - Alcool éthylique | 10 g |
| - Triéthanolamine qs pH = 7 | |
| - Eau déminéralisée | qsp 100 g |

### Composition (B14)

| | |
|---|---|
| - Peroxydase de raifort | 2600 unités |
| - Eau oxygénée à 20 volumes (acidifiée à pH 2 par l'acide orthophosphorique) | 2,5 g |
| - Monoéthanolamine qs pH =5,5 | |
| - Eau déminéralisée | qsp 100 g |

On utilise le même procédé que dans l'exemple 1. Une coloration châtain clair est obtenue sur cheveux initialement blancs.

### EXEMPLE 15

### Composition (A15)

| | |
|---|---|
| - 5-aminoindole | 1 g |
| - Alcool éthylique | 10 g |
| - Eau déminéralisée | qsp 100 g |

### Composition (B15)

| | |
|---|---|
| - Peroxydase de raifort | 3000 unités |
| - Eau oxygénée à 20 volumes (acidifiée à pH 2 par l'acide orthophosphorique) | 2,5 g |
| - Monoéthanolamine qs pH = 5,8 | |
| - Eau déminéralisée | qsp 100 g |

On utilise le même procédé que dans l'exemple 1. Une coloration châtain est obtenue sur cheveux initialement blancs.

### EXEMPLE 16

### Composition (A16)

| | |
|---|---|
| - 4-aminoindole | 0,5 g |
| - Alcool éthylique | 10 g |
| - Triéthanolamine qs pH = 6,5 | |
| - Eau déminéralisée | qsp 100 g |

### Composition (B16)

| | |
|---|---|
| - Peroxydase de raifort | 2600 unités |
| - Eau oxygénée à 20 volumes (acidifiée à pH 2 par l'acide orthophosphorique) | 2,5 g |
| - Monoéthanolamine qs pH = 6 | |
| - Eau déminéralisée | qsp 100 g |

On utilise le même procédé que dans l'exemple 1. Une coloration châtain est obtenue sur cheveux initialement blancs.

### EXEMPLE 17

### Composition (A17)

| | |
|---|---|
| - 1-méthyl 5,6-dihydroxyindole | 1 g |
| - Alcool éthylique | 10 g |
| - Triéthanolamine qs pH = 7 | |
| - Eau déminéralisée | qsp 100 g |

### Composition (B17)

| | |
|---|---|
| - Peroxydase de raifort | 2600 unités |
| - Eau oxygénée à 20 volumes (acidifiée à pH 2 par l'acide orthophosphorique) | 2,5 g |
| - Monoéthanolamine qs pH = 5,9 | |
| - Eau déminéralisée | qsp 100 g |

On utilise le même procédé que dans l'exemple 1. Une coloration châtain est obtenue sur cheveux initialement blancs.

### EXEMPLE 18

Un tubercule de raifort frais est finement broyé grâce à un mixeur, appareil vendu dans le commerce en tant que robot ménager. Le broyat est employé dans la fabrication de la seconde composition de cet exemple.

### Composition (A18)

| | |
|---|---|
| - 5,6-dihydroxyindole | 1 g |
| - Alcool éthylique | 10 g |
| - Eau déminéralisée | qsp 100 g |
| Le pH de la solution est de 6,4. | |

### Composition (B18)

| | |
|---|---|
| - Broyat de raifort frais | 10 g |
| - Eau oxygénée à 20 volumes (acidifiée à pH 2 par l'acide orthophosphorique) | 2,5 g |
| - Monoéthanolamine qs pH = 5,5 | |
| - Eau déminéralisée | qsp 100 g |

La première composition est appliquée sur les cheveux blancs et laissée posée pendant 15 minutes. On rince. On applique la deuxième composition en laissant poser pendant 15 minutes. On rince. On note après shampooing que les cheveux sont de couleur châtain.

### EXEMPLE 19

### Composition (A19)

| | |
|---|---|
| - 5,6-dihydroxyindoline | 1 g |
| - Alcool éthylique | 10 g |
| - Triéthanolamine qs pH = 7 | |
| - Eau déminéralisée | qsp 100 g |

### Composition (B19)

| | |
|---|---|
| - Peroxydase de raifort | 2600 unités |
| - Eau oxygénée à 20 volumes (acidifiée à pH 2 par l'acide orthophosphorique) | 2,5 g |
| - Monoéthanolamine qs pH = 5,9 | |
| - Eau déminéralisée | qsp 100 g |

On utilise le même procédé que dans l'exemple 1. Une coloration châtain est obtenue sur cheveux initialement blancs.

### EXEMPLE 20

### Composition (A20)

| | |
|---|---|
| - 5,6-dihydroxyindole | 1,5 g |
| - Triéthanolamine qs pH = 6,5 | |
| - Ethanol | 10 g |
| - Eau déminéralisée | qsp 100 g |

### Composition (B20)

| | |
|---|---|
| - Glucose | 10 g |
| - Glucose oxydase | 15000 unités |
| - Peroxydase de raifort | 2600 unités |
| - Eau déminéralisée | qsp 100 g |
| Le pH de cette composition est de 6,8. | |

La première composition est appliquée sur une mèche de cheveux, initialement blancs, et laissée posée pendant 15 minutes. On rince. La seconde composition est appliquée et laissée posée pendant 15 minutes. Un rinçage final et un séchage terminent l'opération. La coloration obtenue est châtain clair.

### EXEMPLE 21

### Composition (A21)

### Identique à la composition (A20) de l'exemple 20.

### Composition (B21)

| | |
|---|---|
| - Mélange (80/20) d'alcools cétylstéarylique et cétylstéarylique oxyéthylénés à 33 moles d'oxyde d'éthylène, vendu sous la dénomination LANOL CTO par la Société SEPPIC | 2 g |
| - Glycérine | 0,4 g |
| - Glucose | 10g g |
| - Glucose oxydase | 15000 unités |
| - Peroxydase de raifort | 2600 unités |
| - Acide orthophosphorique qs pH = 7 | |
| - Eau déminéralisée | qsp 100 g |

La première composition est appliquée sur une mèche de cheveux, initialement blancs, et laissée posée pendant 15 minutes. On rince. La seconde composition est appliquée et laissée posée pendant 15 minutes. Un rinçage final et un séchage terminent l'opération. La coloration obtenue est châtain.

### EXEMPLE 22

### Composition (A22)

### Identique à la composition (A20) de l'exemple (20)

### Composition (B22)

| | |
|---|---|
| - Acide lactique | 5 g |
| - Lactate oxydase | 200 unités |
| - Peroxydase de raifort | 2600 unités |
| - Monoéthanolamine qs pH = 7,5 | |
| - Eau déminéralisée | qsp 100 g |

La première composition est appliquée sur des cheveux initialement blancs, et laissée posée pendant 15 minutes. On rince. La seconde composition est appliquée et laissée posée pendant 45 minutes. Un rinçage final et un séchage terminent l'opération. La coloration obtenue est blond foncé.

## Revendications

1. Procédé de teinture des fibres kératiniques, **caractérisé par le fait que** l'on applique sur ces fibres au moins :
- un composant (1) contenant dans un milieu aqueux approprié pour la teinture, au moins un composé indolique ou indolinique de formule : 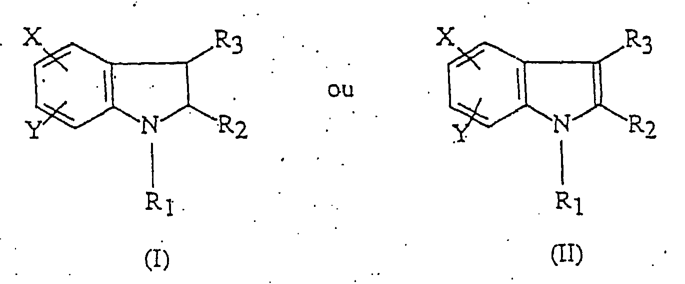
dans lesquelles:
R₁ représente un atome d'hydrogène, un radical alkyle en C₁-C₄;
R₂ représente un hydrogène, un atome d'hydrogène, un radical alkyle en C₁-C₄ ou -COOH;
R₃ représente un atome d'hydrogène ou un radical alkyle en C₁-C₄;
X désigne un atome d'hydrogène. NH₂ OH, un radical alkyle en C₁-C₄ ou un radical alcoxy en C₁-C₄;
Y désigne OH ou NH₂;
sous réserve que lorsque X désigne OH ou alkyle, X occupe les positions 5, 6 ou 7 et Y est en position ortho par rapport au groupe X ;
- un composant (2) contenant dans un milieu aqueux approprié pour la teinture, au moins une peroxydase ;
- un composant (3) contenant dans un milieu, aqueux approprié pour la teinture, au moins du peroxyde d'hydrogène où une source de peroxyde d'hydrogène enzymatique ;
le composant (1) étant appliqué sur les fibres en premier, séparément du composant (3)
dans lequel on applique
dans une première étape une composition contenant dans un milieu aqueux approprié pour la teinture, au moins le composant (1) et éventuellement le composant (2), puis
- soit dans une deuxième étape une composition contenant dans un milieu aqueux approprié pour la teinture au moins les composants (2) et (3), la composition contenant le composant (1) né contenant pas le composant (2),
- soit dans une deuxième étape une composition contenant dans un milieu aqueux approprié pour la teinture le composant (2) et dans une troisième étape une composition contenant dans un milieu aqueux approprié pour la teinture le composant (3), la composition contenant le composant (1) ne contenant pas le composant (2)
- soit dans une deuxième étape une composition contenant dans un milieu aqueux approprié pour la teinture le composant (3) lorsque la composition appliquée dans la première étape contient le composant (2),
le pH des compositions mises en oeuvre variant de 4,5 à 7,5.

2. Procédé selon la revendication 1; **caractérisé par le fait que** le composant (1) contient un composé indolique .ou indolinique choisi parmi le 5,6-dihydroxyindole, le 2-méthyl 5,6-dihydroxyindole, le 3-méthyl 5,6-dihydroxyindole, le 4-hydroxyindole, le 2,3-diméthyl 5,6-dihydroxyindole, le 6-hydroxy 5-méthoxyindole, le 6-hydroxyindole, le 5-hydroxyindole, le 7-hydroxyindole, le 7-arninoindole, le 5-amino indole, l'acide 5,6-dihydroxyindole 2-carboxylique, le 4-arninoiridole, le 1-méthyl 5,6-dihydroxyindole, la 5,6-dihydroxyindoline, la N-méthyl 5,6-dihydroxyindoline, la N-éthyl 5,6-dihydroxyindoline, la N-butyl 5,6-dihydroxyindoline, la 4-hydroxy 5-méthoxyindoline, la 6-hydroxy 7-méthoxyindoline, la 6,7-dihydroxyindoline, la 4,5-dihydroxyindoline, la 5-méthoxy 6-hydroxyindoline et leurs sels.

3. Procédé selon la revendication 1 ou 2, **caractérisé par le fait que** la peroxydase est utilisée à des quantités comprises entre 100 et 40.000 unités pour 100 g de composants (1), (2) et (3) à appliquer sur les fibres.

4. Procédé selon l'une quelconque des revendications 1 à 3, **caractérisé par le fait que** la source de peroxyde d'hydrogène enzymatique est constituée d'un ou plusieurs substrat(s) naturel(s) associé(s) à un (ou des) système(s) enzymatique(s) oxydase(s) catalysant l'oxydation d'une molécule de substrat par une molécule d'oxygène par transfert d'un seul atome d'oxygène.

5. Procédé selon la revendication 4, **caractérisé par le fait que** le système substrat/enzyme d'oxydation par l'oxygène du substrat par transfert d'un seul atome d'oxygène, est choisi parmi les systèmes suivants :
- glucose/glucose oxydase
- galactose/galactose oxydase
- pyranose/pyranose oxydase
- L-sorbose/L-sorbose oxydase
- éthanol/alcool oxydase (1.1.3.13)
- isopropanol/alcool secondaire oxydase (1.1.3.18)
- acide pyruvique/pyruvate oxydase
- acide oxalique/oxalate oxydase
- acide aspartique/aspartate oxydase
- acide glutarnique/L-glutarnate oxydase
- acide urique/uricase
- acide lactique/lactate oxydase,
ou leurs mélanges.

6. Procédé selon la revendication 4 ou 5, **caractérisé par le fait que** les systèmes enzymatiques oxydases sont utilisés dans des quantités comprises entre 100 et 20.000 unités catalytiques d'oxydase pour 100 g de composants (1), (2) et (3) à appliquer sur les fibres kératiniques.

7. Procédé selon l'une quelconque des revendications 1 à 6, **caractérisé par le fait qu'**on applique sur les fibres kératiniques, dans une première étape, une composition (A) contenant dans un milieu aqueux approprié pour la teinture, au moins le composant (1) puis, dans une deuxième étape, une composition (B) contenant, dans un milieu aqueux approprié pour la teinture, au moins les composants (2) et (3), les deux étapes étant éventuellement séparées par un rinçage intermédiaire, le pH des compositions variant de 4,5 à 7,5.

8. Procédé selon la revendication 7, **caractérisé par le fait que** le temps de pose de la composition (A) est de 20 secondes à 20 minutes lorsque le composant (3) contient du peroxyde d'hydrogène ou de 1 minute à 1 heure lorsque le composant (3) contient une source de peroxyde d'hydrogène enzymatique et que le temps de pose de la composition (B) varie de 1 minute à 1 heure.

9. Procédé selon l'une quelconque des revendications 1 à 6, **caractérisé par le fait qu'**on applique sur les fibres dans une première étape, une composition (C) contenant dans un milieu aqueux approprié pour la teinture, au moins les composants (1) et (2) et dans une deuxième étape, une composition (D) contenant dans un milieu aqueux approprié pour la teinture, le composant (3), le pH des compositions variant de 4,5 à 7,5.

10. Procédé selon la revendication 9, **caractérisé par le fait que** le temps de pose de la composition (C) varie de 20 secondes à 20 minutes et que le temps de pose de la composition (D) varie de 1 minute à 1 heure.

11. Procédé selon l'une quelconque des revendications 1 à 6, **caractérisé par le fait que** l'on applique sur les fibres dans une première étape, une composition (E) contenant dans un milieu aqueux approprié pour la teinture, au moins le composant (1), dans une deuxième étape, une composition (F) contenant dans un milieu aqueux approprié pour la teinture, le composant (2) puis, dans une troisième étape, une composition (G) contenant dans un milieu aqueux approprié pour la teinture, le composant (3), le pH des compositions variant de 4,5 à 7,5.

12. Procédé selon la revendication 11, **caractérisé par le fait que** les temps de pose des compositions (E) et (F) varient de 20 secondes à 20 minutes et que le temps de pose de la composition (G) varie de 1 minute à 1 heure.

13. Procédé selon l'une quelconque des revendications 1 à 12, **caractérisé par le fait qu'**il comprend une étape finale de rinçage et de séchage.

14. Procédé selon l'une quelconque des revendications 7 à 13, **caractérisé par le fait que** le composé de formule (I) ou (II) telle que définie dans la revendication 1, est utilisé dans des proportions comprises entre 0,2 et 5% en poids par rapport au poids total de la composition (A), (C) ou (E).

15. Procédé selon l'une quelconque des revendications 7 à 14, **caractérisé par le fait que** le peroxyde d'hydrogène est utilisé dans des proportions comprises entre 0,03 et 10% en poids par rapport au poids total de la composition (B), (D) ou (G).

16. Procédé selon l'une quelconque des revendications 4 à 15, **caractérisé par le fait que** le substrat naturel est présent dans des proportions comprises entre 1 et 25 % en poids par rapport au poids total du ou des composant(s) (2) et/ou (3).

17. Procédé selon l'une quelconque des revendications 7 à 16, **caractérisé par le fait que** la composition (A), (C) ou (E) contient en plus un colorant d'oxydation du type para et/ou ortho et/ou des coupleurs et/ou un colorant direct choisi parmi les dérivés nitrés benzéniques, lés anthraquinones, les naphtoquinones, les benzoquinones et les colorants azoïques.

18. Procédé selon l'une quelconque des revendications 7 à 17, **caractérisé par le fait que** la composition (A), (C) ou (E) contient en plus un solvant choisi parmi les alcools inférieurs, les éthers de glycol, les esters de glycols, les glycols ou les esters inférieurs.

19. Procédé selon l'une quelconque des revendications 7 à 18, **caractérisé par le fait que** l'une au moins des compositions (A) à (G) utilisées contient des adjuvants choisis parmi les agents tensio-actifs anioniques, cationiques, non-ioniques, amphotères ou leurs mélanges, les agents épaississants, les parfums, les agents séquestrants, les agents de traitement, les agents dispersants, les agents de conditionnement, des conservateurs, des agents de gonflement des fibres kératiniques ou leurs mélanges.

## Patentansprüche

1. Verfahren zum Färben von Keratinfasern, **dadurch gekennzeichnet, dass** auf die Fasern zumindest aufgetragen wird:
- eine Komponente (1), die in einem zum Färben geeigneten wässrigen Medium mindestens ein Indol oder Indolin der folgenden Formel enthält: 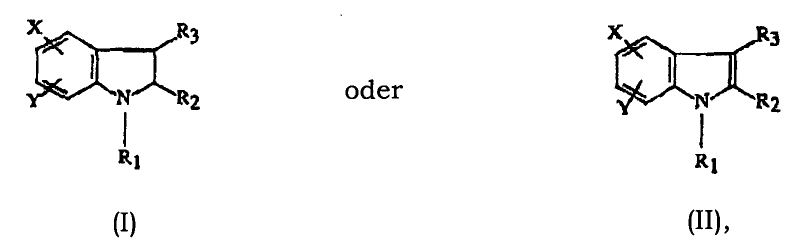 worin bedeuten:
R₁ ein Wasserstoffatom oder eine C₁₋₄-Alkylgruppe;
R₂ ein Wasserstoffatom, eine C₁₋₄-Alkylgruppe oder -COOH;
R₃ ein Wasserstoffatom oder eine C₁₋₄-Alkylgruppe;
X ein Wasserstoffatom, NH₂, OH, C₁₋₄-Alkyl oder C₁₋₄-Alkoxy;
Y OH oder NH₂;
mit der Maßgabe, dass sich X in den Stellungen 5, 6 oder 7 befindet und Y in Bezug auf die Gruppe X in ortho-Stellung substituiert ist, wenn X OH oder Alkyl bedeutet:
- eine Komponente (2), die in einem zum Färben geeigneten wässrigen Medium mindestens eine Peroxidase enthält;
- eine Komponente (3), die in einem zum Färben geeigneten wässrigen Medium zumindest Wasserstoffperoxid oder eine enzymatische Wasserstoffperoxidquelle enthält;
wobei die Komponente (1) getrennt von der Komponente (3) zuerst auf die Fasern aufgetragen wird,
wobei in dem Verfahren in einem ersten Schritt eine Zusammensetzung aufgetragen wird, die in einem zum Färben geeigneten wässrigen Medium mindestens die Komponente (1) und gegebenenfalls die Komponente (2) enthält, und anschließend
- entweder in einem zweiten Schritt eine Komponente, die in einem zum Färben geeigneten wässrigen Medium zumindest die Komponenten (2) und (3) enthält, wobei die Zusammensetzung, die die Komponente (1) enthält, nicht die Komponente (2) enthält,
- oder in einem zweiten Schritt eine Zusammensetzung, die in einem zum Färben geeigneten wässrigen Medium die Komponente (2) enthält, und in einem dritten Schritt eine Zusammensetzung, die in einem zum Färben geeigneten wässrigen Medium die Komponente (3) enthält, wobei die Zusammensetzung, die die Komponente (1) enthält, keine Komponente (2) aufweist,
- oder in einem zweiten Schritt eine Zusammensetzung, die in einem zum Färben geeigneten wässrigen Medium die Komponente (3) enthält, wenn die in dem ersten Schritt aufgetragene Zusammensetzung die Komponente (2) aufweist,
wobei der pH-Wert der verwendeten Zusammensetzungen im Bereich von 4,5 bis 7,5 liegt.

2. Verfahren nach Anspruch 1, **dadurch gekennzeichnet, dass** die Komponente (1) ein Indol oder Indolin enthält, das unter 5,6-Dihydroxyindol, 2-Methyl-5,6-dihydroxyindol, 3-Methyl-5,6-dihydroxyindol, 4-Hydroxyindol, 2,3-Dimethyl-5,6-dihydroxyindol, 6-Hydroxy-5-methoxyindol, 6-Hydroxyindol, 5-Hydroxyindol, 7-Hydroxyindol, 7-Aminoindol, 5-Aminoindol, 5,6-Dihydroxyindol-2-carbonsäure, 4-Aminoindol, 1-Methyl-5,6-dihydroxyindol, 5,6-Dihydroxyindol, N-Methyl-5,6-dihydroxyindolin, N-Ethyl-5,6-dihydroxyindolin, N-Butyl-5,6-dihydroxyindolin, 4-Hydroxy-5-methoxyindolin, 6-Hydroxy-7-methoxyindolin, 6,7-Dihydroxyindolin, 4,5-Dihydroxyindolin, 5-Methoxy-6-hydroxyindolin und deren Salzen ausgewählt ist.

3. Verfahren nach Anspruch 1 oder 2, **dadurch gekennzeichnet, dass** die Peroxidase in Mengenanteilen von 100 bis 40 000 Einheiten auf 100 g der auf die Fasern aufzubringenden Komponenten (1), (2) und (3) verwendet wird.

4. Verfahren nach einem der Ansprüche 1 bis 3, **dadurch gekennzeichnet, dass** die enzymatische Wasserstoffperoxidquelle aus einem oder mehreren natürlichen Substraten in Kombination mit einem (oder mehreren) System(en) enzymatischer Oxidase(n) besteht, wobei die Oxidation eines Substratmoleküls durch ein Sauerstoffmolekül durch Übertragung nur eines Sauerstoffatoms katalysiert wird.

5. Verfahren nach Anspruch 4, **dadurch gekennzeichnet, dass** das System aus Substrat und Enzym für die Oxidation des Substrats mit Sauerstoff durch Transfer nur eines Sauerstoffatoms unter den folgenden Systemen ausgewählt ist:
- Glucose/Glucose-Oxidase
- Glactose/Galactose-Oxidase
- Pyranose/Pyranose-Oxidase
- L-Sorbose/L-Sorbose-Oxidase
- Ethanol/Alkohol-Oxidase (1.1.3.13)
- Isopropanol/Sekundäralkohol-Oxidase (1.1.3.18)
- Brenztraubensäure/Pyruvat-Oxidase
- Oxalsäure / Oxalat-Oxidase
- Asparaginsäure/Aspartat-Oxidase
- Glutaminsäure/L-glutamat-Oxidase
- Harnsäure/Uricase
- Milchsäure/Lactat-Oxidase
oder deren Gemische.

6. Verfahren nach Anspruch 4 oder 5, **dadurch gekennzeichnet, dass** die enzymatischen Oxidasesysteme in Mengen von 100 bis 20 000 katalytischen Einheiten der Oxidase auf 100 g der auf die Keratinfasern aufzubringenden Komponenten (1), (2) und (3) verwendet werden.

7. Verfahren nach einem der Ansprüche 1 bis 6, **dadurch gekennzeichnet, dass** auf die Keratinfasern in einem Schritt eine Zusammensetzung (A), die in einem zum Färben geeigneten wässrigen Medium zumindest die Komponente (1) enthält, und anschließend in einem zweiten Schritt eine Zusammensetzung (B) aufgetragen wird, die in einem zum Färben geeigneten wässrigen Medium zumindest die Komponenten (2) und (3) enthält, wobei zwischen den beiden Schritten gegebenenfalls gespült wird, wobei der pH-Wert der Zusammensetzungen im Bereich von 4,5 bis 7,5 liegt.

8. Verfahren nach Anspruch 7, **dadurch gekennzeichnet, dass** die Einwirkzeit der Komponente (A) 20 Sekunden bis 20 Minuten beträgt, wenn die Komponente (3) Wasserstoffperoxid enthält, oder 1 Minute bis 1 Stunde, wenn die Komponente (3) eine enzymatische Wasserstoffperoxidquelle enthält, und **dadurch**, dass die Einwirkzeit der Zusammensetzung (B) im Bereich von 1 Minute bis 1 Stunde liegt.

9. Verfahren nach einem der Ansprüche 1 bis 6, **dadurch gekennzeichnet, dass** auf die Fasern in einem ersten Schritt eine Zusammensetzung (C), die in einem zum Färben geeigneten wässrigen Medium zumindest die Komponenten (1) und (2) enthält, und in einem zweiten Schritt eine Zusammensetzung (D) aufgetragen wird, die in einem zum Färben geeigneten wässrigen Medium die Komponente (3) enthält, wobei der pH-Wert der Zusammensetzungen im Bereich von 4,5 bis 7,5 liegt.

10. Verfahren nach Anspruch 9, **dadurch gekennzeichnet, dass** die Einwirkzeit der Zusammensetzung (C) im Bereich von 20 Sekunden bis 20 Minuten liegt, und **dadurch**, dass die Einwirkzeit der Zusammensetzung (D) im Bereich von 1 Minute bis 1 Stunde liegt.

11. Verfahren nach einem der Ansprüche 1 bis 6, **dadurch gekennzeichnet, dass** auf die Fasern in einem ersten Schritt eine Zusammensetzung (E), die in einem zum Färben geeigneten wässrigen Medium zumindest die Komponente (1) enthält, in einem zweiten Schritt eine Zusammensetzung (F), die in einem zum Färben geeigneten wässrigen Medium die Komponente (2) enthält, und anschließend in einem dritten Schritt eine Zusammensetzung (G) aufgetragen wird, die in einem zum Färben geeigneten wässrigen Medium die Komponente (3) enthält, wobei der pH-Wert der Zusammensetzungen im Bereich von 4,5 bis 7,5 liegt.

12. Verfahren nach Anspruch 11, **dadurch gekennzeichnet, dass** die Einwirkzeit der Zusammensetzungen (E) und (F) im Bereich von 20 Sekunden bis 20 Minuten liegt, und **dadurch**, dass die Einwirkzeit der Zusammensetzung (G) im Bereich von 1 Minute bis 1 Stunde liegt.

13. Verfahren nach einem der Ansprüche 1 bis 12, **dadurch gekennzeichnet, dass** es am Ende einen Schritt umfasst, in dem gespült und getrocknet wird.

14. Verfahren nach einem der Ansprüche 7 bis 13, **dadurch gekennzeichnet, dass** die in Anspruch 1 definierte Verbindung der Formel (I) oder (II) in Mengenanteilen von 0,2 bis 5 Gew.-%, bezogen auf das Gesamtgewicht der Zusammensetzung (A), (C) oder (E) verwendet wird.

15. Verfahren nach einem der Ansprüche 7 bis 14, **dadurch gekennzeichnet, dass** das Wasserstoffperoxid in Mengenanteilen von 0,03 bis 10 Gew.-%, bezogen auf das Gesamtgewicht der Zusammensetzung (B), (D) oder (G) verwendet wird.

16. Verfahren nach einem der Ansprüche 4 bis 15, **dadurch gekennzeichnet, dass** das natürliche Substrat in Mengenanteilen von 1 bis 25 Gew.-%, bezogen auf das Gesamtgewicht der Komponente(n) (2) und/oder (3), vorliegt.

17. Verfahren nach einem der Ansprüche 7 bis 16, **dadurch gekennzeichnet, dass** die Zusammensetzung (A), (C) oder (E) ferner einen Oxidationsfarbstoff vom para-Typ und/oder ortho-Typ und/oder Kuppler und/oder einen Direktfarbstoff, der unten der nitrierten Benzolderivaten, Anthrachinonen, Naphthochinonen, Benzochinonen und Azofarbstoffen ausgewählt ist, enthält.

18. Verfahren nach einem der Ansprüche 7 bis 17, **dadurch gekennzeichnet, dass** die Zusammensetzung (A), (C) oder (E) ferner ein Lösungsmittel enthält, das unter den niederen Alkoholen, Glycolethern, Glycolestern, Glycolen oder niederen Estern ausgewählt ist.

19. Verfahren nach einem der Ansprüche 7 bis 18, **dadurch gekennzeichnet, dass** zumindest eine der Zusammensetzungen (A) bis (G) Zusatzstoffe enthält, die unter den anionischen, kationischen, nichtionischen, amphoteren grenzflächenaktiven Stoffen oder deren Gemischen, Verdickungsmitteln, Parfums, Maskierungsmitteln, Behandlungsmitteln, Dispergiermitteln, Konditioniermitteln, Konservierungsmitteln, Quellmitteln für Keratinfasern oder deren Gemischen ausgewählt ist.

## Claims

1. Process for dyeing keratinous fibres, **characterized in that** at least:
- a component (1) comprising, in an aqueous medium appropriate for dyeing, at least one indole or indoline compound of formula: 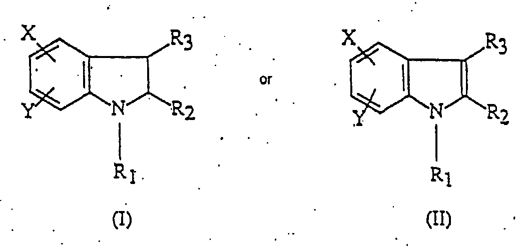 in which:
R₁ represents a hydrogen atom or a C₁-C₄ alkyl radical;
R₂ represents a hydrogen atom, a C₁-C₄ alkyl radical or -COOH;
R₃ represents a hydrogen atom or a C₁-C₄ alkyl radical;
X denotes a hydrogen atom, NH₂, OH, a C₁-C₄ alkyl radical or a C₁-C₄ alkoxy radical;
Y denotes OH or NH₂;
with the proviso that, when X denotes OH or alkyl, X occupies the 5, 6 or 7 position and Y is in the ortho position with respect to the X group;
- a component (2) comprising, in an aqueous medium appropriate for dyeing, at least one peroxidase;
- a component (3) comprising, in an aqueous medium appropriate for dyeing, at least hydrogen peroxide or one source of enzymatic hydrogen peroxide;
are applied to these fibres;
the component (1) being applied to the fibres first, separately from the component (3),
in which process:
in a first stage, a composition comprising, in an aqueous medium appropriate for dyeing, at least the component (1) and optionally the component (2) is applied, then
- either, in a second stage, a composition comprising, in an aqueous medium appropriate for dyeing, at least the components (2) and (3) is applied, the composition comprising the component (1) not comprising the component (2),
- or, in a second stage, a composition comprising, in an aqueous medium appropriate for dyeing, the component (2) is applied and, in a third stage, a composition comprising, in an aqueous medium appropriate for dyeing, the component (3) is applied, the composition comprising the component (1) not comprising the component (2),
- or, in a second stage, a composition comprising, in an aqueous medium appropriate for dyeing, the component (3) is applied when the composition applied in the first stage comprises the component (2),
the pH of the compositions employed varying from 4.5 to 7.5.

2. Process according to Claim 1, **characterized in that** the component (1) comprises an indole or indoline compound chosen from 5,6-dihydroxyindole, 2-methyl-5,6-dihydroxyindole, 3-methyl-5,6-dihydroxyindole, 4-hydroxyindole, 2,3-dimethyl-5,6-dihydroxyindole, 6-hydroxy-5-methoxyindole, 6-hydroxyindole, 5-hydroxyindole, 7-hydroxyindole, 7-aminoindole, 5-aminoindole, 5,6-dihydroxyindole-2-carboxylic acid, 4-aminoindole, 1-methyl-5,6-dihydroxyindole, 5,6-dihydroxyindoline, N-methyl-5,6-dihydroxyindoline, N-ethyl-5,6-dihydroxyindoline, N-butyl-5,6-dihydroxyindoline, 4-hydroxy-5-methoxyindoline, 6-hydroxy-7-methoxyindoline, 6,7-dihydroxyindoline, 4,5-dihydroxyindoline, 5-methoxy-6-hydroxyindoline and their salts.

3. Process according to Claim 1 or 2, **characterized in that** the peroxidase is used at amounts of between 100 and 40 000 units per 100 g of components (1), (2) and (3) to be applied to the fibres.

4. Process according to any one of Claims 1 to 3, **characterized in that** the source of enzymatic hydrogen peroxide is composed of one or more natural substrate(s) in association with one (or more) oxidase enzymatic system(s) which catalyse(s) the oxidation of a substrate molecule by an oxygen molecule by transfer of a single oxygen atom.

5. Process according to Claim 4, **characterized in that** the substrate/enzyme system for oxidation by oxygen of the substrate by transfer of a single oxygen atom is chosen from the following systems:
- glucose/glucose oxidase
- galactose/galactose oxidase
- pyranose/pyranose oxidase
- L-sorbose/L-sorbose oxidase
- ethanol/alcohol oxidase (1.1.3.13)
- isopropanol/secondary alcohol oxidase (1.1.3.18)
- pyruvic acid/pyruvate oxidase
- oxalic acid/oxalate oxidase
- aspartic acid/aspartate oxidase
- glutamic acid/L-glutamate oxidase
- uric acid/uricase
- lactic acid/lactate oxidase
or their mixtures.

6. Process according to Claim 4 or 5, **characterized in that** the oxidase enzymatic systems are used in amounts of between 100 and 20 000 oxidase catalytic units per 100 g of components (1), (2) and (3) to be applied to the keratinous fibres.

7. Process according to any one of Claims 1 to 6, **characterized in that**, in a first stage, a composition (A) comprising, in an aqueous medium appropriate for dyeing, at least the component (1) is applied to the keratinous fibres and then, in a second stage, a composition (B) comprising, in an aqueous medium appropriate for dyeing, at least the components (2) and (3) is applied to the keratinous fibres, the two stages optionally being separated by an intermediate rinsing, the pH of the compositions varying from 4.5 to 7.5.

8. Process according to Claim 7, **characterized in that** the leave-in time of the composition (A) is from 20 seconds to 20 minutes when the component (3) comprises hydrogen peroxide or from 1 minute to 1 hour when the component (3) comprises a source of enzymatic hydrogen peroxide and that the leave-in time for the composition (B) varies from 1 minute to 1 hour.

9. Process according to any one of Claims 1 to 6, **characterized in that**, in a first stage, a composition (C) comprising, in an aqueous medium appropriate for dyeing, at least the components (1) and (2) is applied to the fibres and, in a second stage, a composition (D) comprising, in an aqueous medium appropriate for dyeing, the component (3) is applied to the fibres, the pH of the compositions varying from 4.5 to 7.5.

10. Process according to Claim 9, **characterized in that** the leave-in time of the composition (C) varies from 20 seconds to 20 minutes and that the leave-in time of the composition (D) varies from 1 minute to 1 hour.

11. Process according to any one of Claims 1 to 6, **characterized in that**, in a first stage, a composition (E) comprising, in an aqueous medium appropriate for dyeing, at least the component (1) is applied to the fibres, in a second stage, a composition (F) comprising, in an aqueous medium appropriate for dyeing, the component (2) is applied to the fibres and then, in a third stage, a composition (G) comprising, in an aqueous medium appropriate for dyeing, the component (3) is applied to the fibres, the pH of the compositions varying from 4.5 to 7.5.

12. Process according to Claim 11, **characterized in that** the leave-in times of the compositions (E) and (F) vary from 20 seconds to 20 minutes and that the leave-in time of the composition (G) varies from 1 minute to 1 hour.

13. Process according to any one of Claims 1 to 12, **characterized in that** it comprises a final stage of rinsing and of drying.

14. Process according to any one of Claims 7 to 13, **characterized in that** the compound of formula (I) or (II) as defined in Claim 1 is used in proportions of between 0.2 and 5% by weight, with respect to the total weight of the composition (A), (C) or (E).

15. Process according to any one of Claims 7 to 14, **characterized in that** the hydrogen peroxide is used in proportions of between 0.03 and 10% by weight, with respect to the total weight of the composition (B), (D) or (G).

16. Process according to any one of Claims 4 to 15, **characterized in that** the natural substrate is present in proportions of between 1 and 25% by weight, with respect to the total weight of the component(s) (2) and/or (3).

17. Process according to any one of Claims 7 to 16, **characterized in that** the composition (A), (C) or (E) furthermore comprises an oxidation dye of the para and/or ortho type and/or couplers and/or a direct dye chosen from nitrobenzene derivatives, anthraquinones, naphthoquinones, benzoquinones and azo dyes.

18. Process according to any one of Claims 7 to 17, **characterized in that** the composition (A), (C) or (E) furthermore comprises a solvent chosen from lower alcohols, glycol ethers, glycol esters, glycols or lower esters.

19. Process according to any one of Claims 7 to 18, **characterized in that** at least one of the compositions (A) to (G) used comprises adjuvants chosen from anionic, cationic, nonionic or amphoteric surface-active agents or their mixtures, thickening agents, fragrances, sequestering agents, treatment agents, dispersing agents, conditioning agents, preservatives, agents for expanding keratinous fibres or their mixtures.
